# DEMANDE DE BREVET EUROPEEN

(11) **EP 0 820 764 A1**
(43) Date de publication de la demande: **28.01.1998**
(21) Numéro de dépôt: 97401558.8
(22) Date de dépôt: 02.07.1997
(51) Int. Cl.: A61K 7/48

(54) **Composition comprenant un système polymérique et utilisation dudit système notamment en cosmétique**

(30) Priorité: 24.07.1996 FR 9609319
(71) Demandeur: L'OREAL, 75008 Paris (FR)
(72) Inventeur: De la Poterie, Valérie, 77820 Le Chatelet En Brie (FR)
(74) Mandataire: Dodin, Catherine

(57) **Abrégé**

La présente demande concerne une composition susceptible d'être appliquée sur la peau, les semi-muqueuses et/ou les muqueuses, comprenant un système polymérique qui comprend une dispersion aqueuse de particules de polymère filmogène, ledit système polymérique permettant l'obtention d'un film souple et flexible.

L'invention concerne également l'utilisation d'un tel système polymérique dans une telle composition notamment cosmétique.

## Description

La présente invention a trait à une composition notamment cosmétique susceptible d'être appliquée sur la peau, les semi-muqueuses et/ou les muqueuses. Ladite composition comprend en particulier une dispersion aqueuse de particules de polymère filmogène et peut être utilisée en tant que produit de maquillage.

Les compositions à appliquer sur la peau, les semi-muqueuses et/ou les muqueuses, de type rouge à lèvres ou fond de teint, se présentent généralement sous forme de stick, de pâte souple ou de pâte coulée, et comprennent des corps gras tels que des huiles, des composés pâteux et/ou des cires, et une phase particulaire généralement composée de charges et de pigments.

Ces compositions, lorsqu'elles sont appliquées sur ledit support, présentent toutefois l'inconvénient de transférer. On entend par là le fait que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support.

Il s'en suit donc une persistance médiocre de la composition sur la peau, les semi-muqueuses ou les muqueuses, et la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. On a encore constaté que les eye-liners pouvaient également couler. Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé des compositions de rouge à lèvres 'sans transfert' contenant de 1 à 70% en poids de résine liquide de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).

On connaît également des rouges à lèvres 'sans transfert' contenant une silicone volatile et une résine de silicone comportant une chaîne estérifiée pendante ayant au moins 12 atomes de carbone. Le film de rouge à lèvres présente notamment l'inconvénient de manquer de confort à l'application, en particulier d'être trop sec. Ainsi, d'une manière générale, l'association d'huiles volatiles avec certains composés siliconés permet d'obtenir un résultat 'sans transfert' satisfaisant. Toutefois, les films obtenus après application de ces compositions et évaporation des volatils présentent néanmoins l'inconvénient d'être relativement mats, et conduisent ainsi à un maquillage peu brillant.

Il subsiste donc le besoin d'une composition cosmétique qui transfère peu ou pas du tout, c'est-à-dire d'une composition 'sans transfert', tout en possédant de bonnes propriétés cosmétiques, et en particulier permettant l'obtention d'un film qui peut être, au choix, plus ou moins brillant.

La présente invention a pour but de proposer une composition qui permet d'obtenir un film de très bonne tenue, qui ne transfère pas et ne tache pas un support avec lequel il serait en contact, et qui ne migre pas au cours du temps, tout en permettant d'obtenir un maquillage et/ou un film brillant.

Ainsi, un objet de l'invention est l'utilisation dans une composition susceptible d'être appliquée sur la peau, les semi-muqueuses et/ou les muqueuses, d'un système polymérique comprenant une dispersion aqueuse de particules de polymère filmogène, ledit système polymérique permettant l'obtention d'un film ayant un module de Young inférieur à environ 200MPa.

Un autre objet de l'invention est l'utilisation pour maquiller, protéger et/ou traiter non thérapeutiquement la peau, les semi-muqueuses et/ou les muqueuses, en particulier les lèvres du visage, dudit système polymérique ou d'une composition le comprenant.

Un autre objet est l'utilisation pour la fabrication d'une composition destinée à traiter thérapeutiquement la peau, les semi-muqueuses et/ou les muqueuses, en particulier les lèvres du visage, dudit système polymérique ou d'une composition le comprenant.

Un autre objet est une composition susceptible d'être appliquée sur la peau, les semi-muqueuses et/ou les muqueuses, notamment une composition de rouge à lèvres sans transfert, comprenant un système polymérique qui comprend une dispersion aqueuse de particules de polymère filmogène, ledit système polymérique permettant l'obtention d'un film ayant un module de Young inférieur à environ 200 MPa.

On a constaté que la composition selon l'invention est facilement applicable et s'étale aisément et uniformément sur la peau, les semi-muqueuses et les muqueuses, en particulier sur les lèvres du visage.

La composition selon l'invention trouve notamment une application particulièrement intéressante dans le domaine du soin et/ou du maquillage de la peau, des muqueuses et/ou des semi-muqueuses. On entend notamment par muqueuse, la partie interne de la paupière inférieure; parmi les semi-muqueuses, on entend plus particulièrement les lèvres du visage.

La composition selon l'invention permet l'obtention d'un film homogène, qui présente une texture légère et reste confortable à porter tout au long de la journée. Le film n'est pas du tout collant, tout en étant mou, souple, élastique et flexible sur la peau; il suit les mouvements du support sur lequel il est déposé, sans se craqueler et/ou se décoller. Il adhère notamment parfaitement sur les lèvres du visage.
La composition selon l'invention trouve une application toute particulière dans le domaine des produits de maquillage des lèvres du visage, notamment en tant que rouge à lèvres. Elle trouve également une autre application avantageuse dans le domaine des eye-liners.
D'autre part, le film obtenu peut être très brillant, ou plus ou moins mat, selon la nature des constituants de la composition, d'où une gamme plus étendue de produits de maquillage, brillants ou mats, au choix.

La composition selon l'invention comprend donc un système polymérique qui comprend au moins une dispersion aqueuse de particules de polymère filmogène. Parmi les polymères filmogènes utilisables dans le cadre de la présente invention, on peut citer les polymères synthétiques, de type polycondensat ou de type radicalaires, les polymères d'origine naturelle, et leurs mélanges.

On peut ainsi citer, parmi les polycondensats, les polyuréthannes anioniques, cationiques, non ioniques ou amphotères, les polyuréthannes-acryliques, les polyuréthannes-polyvinylpirrolidones, les polyester-polyuréthannes, les polyétherpolyuréthannes, les polyurées, les polyurée/polyuréthannes, et leurs mélanges.
Le polyuréthanne peut être, par exemple, un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.

Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

On peut également citer les polyesters, les polyesters amides, les polyesters à chaîne grasse, les polyamides, et les résines époxyesters.

Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylène diamine, l'hexaméthylènediamine, la méta- ou para-phénylène diamine. Comme aminoalcool, on peut utiliser la monoéthanolamine.

Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzènedicarboxylique.

Les polyesters à chaîne grasse peuvent être obtenus par l'utilisation de diols à chaîne grasse lors de la polycondensation.

Les résines époxyesters peuvent être obtenues par polycondensation d'acides gras avec un condensat aux extrémités α, ω - diépoxy.

Les polymères de type radicalaires peuvent être notamment des polymères, ou des copolymères, acryliques et/ou vinyliques. On utilise de préférence des polymères radicalaires anioniques.

Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.

Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.

On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.

On peut également utiliser des copolymères acryliques/silicones, ou encore des copolymères nitrocellulose/acryliques.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion aqueuse comprenant un ou plusieurs polymères filmogènes peut être préparée par l'homme du métier sur base de ses connaissances générales. Afin d'améliorer le caractère filmogène d'un polymère, par exemple en abaissant sa température de transition vitreuse, il est possible d'ajouter à la dispersion un agent de coalescence, qui peut être choisi parmi les agents de coalescence connus. Dans la présente description, on entend par'dispersion de polymère filmogène', une dispersion susceptible de former un film, comprenant ou ne comprenant pas d'agent de coalescence.

La teneur en matière sèche desdites dispersions aqueuses selon la présente invention peut être de l'ordre de 5-60% en poids, et de préférence 30-40%.

La composition peut comprendre 1-60% en poids, de préférence 5-40% en poids de matière sèche de polymères filmogènes.

La taille des particules de polymères en dispersion aqueuse peut être comprise entre 10-500 nm, et est de préférence comprise entre 20 et 150 nm, ce qui permet d'obtenir un film ayant une brillance remarquable.

Afin de réaliser la présente invention, il est donc nécessaire que le système polymérique permette l'obtention d'un film sur le support sur lequel il est déposé. Ledit film doit avoir, dans les conditions de mesure définies avant les exemples, un module de Young inférieur à environ 200 MPa, de préférence inférieur à environ 100 MPa, et préférentiellement inférieur à 80 MPa.

D'autre part, dans un mode de réalisation préféré, ledit système polymérique peut être choisi de manière à permettre l'obtention d'un film ayant
. une élongation supérieure à environ 200%, et, de manière préférentielle, supérieure à 300%, et/ou
. une dureté inférieure à 110, de préférence inférieure à 70, plus préférentiellement inférieure à 55.

Les méthodes de mesure d'élongation, de dureté et du module de Young (module d'élasticité) sont décrites avant les exemples.

Afin d'obtenir le module de Young, et éventuellement la dureté et/ou l'élongation, souhaités, le système polymérique selon l'invention peut comprendre, en plus de la dispersion aqueuse de particules de polymère filmogène, un composé susceptible d'influencer cette ou ces caractéristiques, à savoir un agent plastifiant. Ledit agent plastifiant peut être choisi parmi tous les composés connus de l'homme du métier comme étant susceptibles de remplir la fonction recherchée. Cet agent peut être hydrosoluble ou insoluble dans l'eau et peut éventuellement se présenter sous forme de dispersion aqueuse.

En particulier, on peut citer, seuls ou en mélange, les plastifiants usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ou en dispersion aqueuse, ayant une température de transition vitreuse faible, inférieure à 25°C, de préférence inférieure à 15°C.

La quantité d'agent plastifiant peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

La composition peut en outre comprendre au moins un colorant hydrosoluble et/ou au moins un pigment, utilisés de manière usuelle dans le domaine de la cosmétique et du maquillage. Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium. Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

On peut également ajouter dans la composition selon l'invention tout additif connu tel que des agents épaississants, par exemple des argiles, des gommes, des silices, les dérivés cellulosiques, un polymère synthétique tel qu'un polymère acrylique ou un polymère associatif de type polyuréthanne; une gomme naturelle telle que la gomme xanthane; des agents d'étalement; des dispersants; des conservateurs; des agents antimousses; des agents mouillants; des filtres UV; des parfums; des charges; des actifs cosmétiques ou pharmaceutiques; des hydratants; des vitamines et leurs dérivés; des matières biologiques et leurs dérivés. Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Le pH de la composition finale obtenue est de préférence inférieur à 9. Cette composition doit bien entendu être apte à se déposer sur un support tel que la peau, les muqueuses et/ou les semi-muqueuses.

La composition selon l'invention peut se présenter sous forme fluide, gélifiée, semi-solide, pâte souple, voire solide telle que de stick ou bâton.
Elle trouve en particulier une application en tant que produit de maquillage, notamment en tant que rouge à lèvres, fond de teint, fard à joues ou fard à paupières, ou encore eye-liner. On peut également envisager une application dans le domaine des compositions de soin, des compositions solaires ou autobronzantes, des compositions dermatologiques ou encore des compositions pharmaceutiques à appliquer sur la peau, les semi-muqueuses et/ou les muqueuses.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### C/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.
Le film déposé sur le support doit avoir une épaisseur d'environ 300 microns avant séchage. Après séchage pendant 7 jours à 21°C et sous une humidité relative de 50%, on obtient un film ayant une épaisseur d'environ 100 microns. Les échantillons mesurés ont une largeur de 5 mm et une épaisseur de 100 microns. La distance entre les mors est de 25 mm. La vitesse de traction est de 1000 mm par minute.
Dans le cadre de l'exemple 1, on effectue également une mesure pour une vitesse de traction de 10 mm par minute.

### Exemple 1

On prépare des dispersions aqueuses comprenant différents polymères. On dépose la composition sur un support de manière à former un film. On mesure le module de Young du film obtenu et l'on apprécie la tenue du film sur les lèvres. Le module de Young est mesuré pour deux vitesses : 10 mm/minute et 1000 mm par minute.

On obtient les résultats suivants :

| Polymère | Module de Young (MPa) 1000 mm / min | Module de Young (MPa) 10 mm / min | Appréciation visuelle |
|---|---|---|---|
| Polyuréthanne 1 SANCURE 2060 | 434 | 553 | se craquèle très vite au milieu des lèvres; se décolle sur les côtés |
| Polyuréthanne 2 SANCURE 815 | 370 | 501 | se craquèle au milieu; se décolle après quelque temps sur les cotés |
| Polyuréthanne 3 SANCURE 898 | 217 | 341 | se craquèle un peu au milieu; se décolle |
| Polyuréthanne 4 NEOREZ R981 | 168,1 | --- | long à craqueler; ne se décolle pas sur les cotés |
| Polyuréthanne 5 SANCURE 878 | 75,1 | 46,9 | très long à craqueler; ne se décolle pas |
| Polyuréthanne 6 SANCURE 861 | 44,3 | 16,1 | ne se craquèle pas; ne se décolle pas; très souple |
| Polyuréthanne 7 SANCURE 2255 | 5,5 | 9,8 | long à craqueler; ne se décolle pas sur les cotés |
| Les polyuréthannes 1, 2 et 4 sont des polyuréthannes/polyesters. | | | |
| Les polyuréthannes 5 et 6 sont des polyuréthannes/polyéthers. | | | |

On constate donc que l'on obtient un film adéquat, ayant une bonne tenue et relativement souple, lorsque le polymère permet l'obtention d'un film ayant un module de Young inférieur à environ 200 MPa, mesuré à une vitesse de 1000 mm par minute. Ceci correspond à un module inférieur à environ 340 MPa mesuré pour une vitesse de 10 mm / minute.

### Exemple 2

On prépare un eye-liner ayant la composition suivante :
. dispersion aqueuse de polyuréthanne (module de Young : 44,3 MPa à 1000 mm/min) 95 g
. pigment 2 g
. agent plastifiant (glycérine) 1,25 g
On obtient une composition facile à appliquer sur le contour de l'oeil, qui donne un trait satiné et qui ne transfert pas et ne coule pas.

### Exemple 3

On prépare un rouge à lèvres ayant la composition suivante :
. dispersion aqueuse de polyuréthanne (module de Young : 75 MPa à 1000 mm/min) 95 g
. pigment 1g
. agent plastifiant (glycérine) 1,25 g

On obtient une composition facile à appliquer sur les lèvres; le film obtenu est très brillant; il ne transfert pas et ne migre pas dans les ridules; il résiste bien et suit le mouvement des lèvres.

### Exemple 4 contre-exemple

On prépare un rouge à lèvres ayant la composition suivante :
. dispersion aqueuse de polyuréthanne (module de Young = 434 MPa à 1000 mm/min) 95 g
. pigment 1g
. agent plastifiant (glycérine) 1,25 g

On obtient un film qui craquèle très rapidement après son application sur les lèvres.

## Revendications

1. Utilisation dans une composition susceptible d'être appliquée sur la peau, les semi-muqueuses et/ou les muqueuses, d'un système polymérique comprenant une dispersion aqueuse de particules de polymère filmogène, ledit système polymérique permettant l'obtention d'un film ayant un module de Young inférieur à environ 200 MPa.

2. Utilisation pour maquiller, protéger et/ou traiter non thérapeutiquement la peau, les semi-muqueuses et/ou les muqueuses, en particulier les lèvres du visage, d'un système polymérique ou d'une composition le comprenant, ledit système comprenant une dispersion aqueuse de particules de polymère filmogène et permettant l'obtention d'un film ayant un module de Young inférieur à environ 200 MPa.

3. Utilisation pour la fabrication d'une composition destinée à traiter thérapeutiquement la peau, les semi-muqueuses et/ou les muqueuses, en particulier les lèvres du visage, d'un système polymérique ou d'une composition le comprenant, ledit système comprenant une dispersion aqueuse de particules de polymère filmogène et permettant l'obtention d'un film ayant un module de Young inférieur à environ 200 MPa.

4. Utilisation selon l'une des revendications précédentes, afin d'obtenir un film de très bonne tenue et/ou qui ne transfère pas et/ou qui ne migre pas et/ou qui ne tache pas.

5. Utilisation selon l'une des revendications précédentes, afin d'obtenir un film souple et/ou élastique et/ou flexible sur la peau et/ou un film qui suit les mouvements de la peau et/ou ne se craquèle pas et/ou ne se décolle pas.

6. Utilisation selon l'une des revendications précédentes, afin d'obtenir un film brillant.

7. Utilisation selon l'une des revendications précédentes, dans laquelle le polymère filmogène est choisi parmi les polyuréthannes, les polyuréthannesacryliques, les polyuréthannes-polyvinylpirrolidones, les polyurées, les polyuréepolyuréthannes, les polyester-polyuréthannes, les polyéther-polyuréthannes, les polyesters, les polyesters amides, les polyesters à chaîne grasse, les polyamides, les résines époxyesters; les polymères, ou copolymères, acryliques et/ou vinyliques; les copolymères acryliques-silicone, les copolymères nitrocellulose-acryliques; les polymères d'origine naturelle, éventuellement modifiés; les dérivés cellulosiques; les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes; et leurs mélanges.

8. Utilisation selon l'une des revendications précédentes, dans laquelle les particules de polymères en dispersion aqueuse ont une taille comprise entre 10-500 nm, de préférence comprise entre 20 et 150 nm.

9. Utilisation selon l'une des revendications précédentes, dans laquelle la composition comprend 1-60% en poids, de préférence 5-40% en poids de matière sèche de polymère filmogène.

10. Utilisation selon l'une des revendications précédentes, dans laquelle le système polymérique permet l'obtention d'un film ayant un module de Young inférieur à environ 100 MPa, de préférence inférieur à environ 80 MPa.

11. Utilisation selon l'une des revendications précédentes, dans laquelle le système polymérique permet l'obtention d'un film ayant une élongation supérieure à environ 200%, de préférence supérieure à 300%, et/ou ayant une dureté inférieure à 110, de préférence inférieure à 70, plus préférentiellement inférieure à 55.

12. Utilisation selon l'une des revendications précédentes, dans laquelle le système polymérique comprend en outre un agent plastifiant.

13. Utilisation selon la revendication 12, dans laquelle l'agent plastifiant est choisi parmi les glycols et leurs dérivés; les esters de glycérol; les dérivés de propylène glycol; des esters d'acides tels que des citrates, des phtalates, des adipates, des carbonates, des tartrates, des phosphates, des sébaçates; des dérivés oxyéthylénés tels que des huiles; des polymères hydrosolubles ou en dispersion aqueuse, ayant une température de transition vitreuse faible, inférieure à 25°C, de préférence inférieure à 15°C; et leurs mélanges.

14. Utilisation selon l'une des revendications précédentes, dans laquelle la composition comprend en outre au moins un colorant hydrosoluble et/ou au moins un pigment.

15. Utilisation selon la revendication 14, dans laquelle le pigment est présent à raison de 0-20% en poids dans la composition finale, de préférence à raison de 1-5% en poids.

16. Utilisation selon l'une des revendications 14 à 15, dans laquelle le pigment peut être choisi parmi les oxydes de titane, de zirconium ou de cérium, les oxydes de zinc, de fer ou de chrome, le bleu ferrique, le noir de carbone, les laques de baryum, strontium, calcium, aluminium; et leurs nanopigments.

17. Utilisation selon la revendication 14, dans laquelle le colorant hydrosoluble est choisi parmi le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

18. Utilisation selon l'une des revendications précédentes, dans un produit de maquillage tel qu'un rouge à lèvres, un fond de teint, un fard à joues ou fard à paupières, un eye-liner; dans une composition de soin; dans une composition solaire; dans une composition autobronzante.

19. Composition susceptible d'être appliquée sur la peau, les semi-muqueuses et/ou les muqueuses, comprenant un système polymérique qui comprend une dispersion aqueuse de particules de polymère filmogène, ledit système polymérique permettant l'obtention d'un film ayant un module de Young inférieur à environ 200 MPa.

20. Composition selon la revendication 19, ladite composition permettant après application l'obtention d'un film de très bonne tenue et/ou qui ne transfère pas et/ou qui ne migre pas et/ou qui ne tache pas.

21. Composition selon l'une des revendications 19 à 20, ladite composition permettant après application l'obtention d'un film brillant.

22. Composition selon l'une des revendications 19 à 21, dans laquelle le polymère filmogène est choisi parmi les polyuréthannes, les polyuréthannes-acryliques, les polyuréthannes-polyvinylpirrolidones, les polyurées, les polyurée-polyuréthannes, les polyester-polyuréthannes, les polyéther-polyuréthannes, les polyesters, les polyesters amides, les polyesters à chaîne grasse, les polyamides, les résines époxyesters; les polymères, ou copolymères, acryliques et/ou vinyliques; les co-polymères acryliques-silicone, les copolymères nitrocellulose-acryliques; les polymères d'origine naturelle, éventuellement modifiés; les dérivés cellulosiques; les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes; et leurs mélanges.

23. Composition selon l'une des revendications 19 à 22, dans laquelle les particules de polymère en dispersion aqueuse ont une taille comprise entre 10-500 nm, de préférence comprise entre 20 et 150 nm.

24. Composition selon l'une des revendications 19 à 23, comprenant 1-60% en poids, de préférence 5-40% en poids de matière sèche de polymère filmogène.

25. Composition selon l'une des revendications 19 à 24, dans laquelle le système polymérique permet l'obtention d'un film ayant un module de Young inférieur à environ 100 MPa, de préférence inférieur à environ 80 MPa.

26. Composition selon l'une des revendications 19 à 25, dans laquelle le système polymérique permet l'obtention d'un film ayant une élongation supérieure à environ 200%, de préférence supérieure à 300%, et/ou ayant une dureté inférieure à 110, de préférence inférieure à 70, plus préférentiellement inférieure à 55.

27. Composition selon l'une des revendications 19 à 26, dans laquelle le système polymérique comprend en outre un agent plastifiant.

28. Composition selon la revendication 27, dans laquelle l'agent plastifiant est choisi parmi les glycols et leurs dérivés; les esters de glycérol; les dérivés de propylène glycol; des esters d'acides tels que des citrates, des phtalates, des adipates, des carbonates, des tartrates, des phosphates, des sébaçates; des dérivés oxyéthylénés tels que des huiles; des polymères hydrosolubles ou en dispersion aqueuse, ayant une température de transition vitreuse faible, inférieure à 25°C, de préférence inférieure à 15°C; et leurs mélanges.

29. Composition selon l'une des revendications 19 à 28, comprenant en outre au moins un colorant hydrosoluble et/ou au moins un pigment.

30. Composition selon la revendication 29, dans laquelle les pigments sont présents à raison de 0-20% en poids, et de préférence 1-5% en poids.

31. Composition selon l'une des revendications 29 à 30, dans laquelle les pigments sont choisis parmi les oxydes de titane, de zirconium ou de cérium, les oxydes de zinc, de fer ou de chrome, le bleu ferrique, le noir de carbone, les laques de baryum, strontium, calcium, aluminium; et leurs nanopigments.

32. Composition selon la revendication 29, dans laquelle le colorant hydrosoluble est choisi parmi le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

33. Composition selon l'une des revendications 19 à 32, destinée à maquiller, protéger et/ou traiter la peau, les semi-muqueuses, et/ou les muqueuses.

34. Composition selon l'une des revendications 19 à 33, se présentant sous la forme d'un produit de maquillage tel qu'un rouge à lèvres, un fond de teint, un fard à joues ou fard à paupières, ou un eye-liner.

35. Composition selon l'une des revendications 19 à 33, se présentant sous la forme d'une composition de soin, d'une composition dermatologique, d'une composition pharmaceutique, d'une composition solaire, d'une composition autobronzante, à appliquer sur la peau, les semi-muqueuses et/ou les muqueuses.

36. Composition de rouge à lèvres sans transfert comprenant un système polymérique qui comprend une dispersion aqueuse de particules de polymère filmogène, ledit système polymérique permettant l'obtention d'un film ayant un module de Young inférieur à environ 200 MPa.
